# EUROPÄISCHE PATENTANMELDUNG

(11) **EP 2 298 414 A1**
(43) Veröffentlichungstag der Anmeldung: **23.03.2011**
(21) Anmeldenummer: 10177404.0
(22) Anmeldetag: 17.09.2010
(51) Int. Cl.: A61N 5/06

(54) **Verfahren und Gerät zum Aktivieren der Selbstheilungskräfte bei Patienten, sowie Verwendungen hierfür**

(30) Priorität: 21.09.2009 DE 102009044061
(71) Anmelder: Teupe, Sigrid, 46282 Dorsten (DE)
(72) Erfinder: Teupe, Sigrid, 46282 Dorsten (DE)
(74) Vertreter: Benedum, Ulrich Max

(57) **Zusammenfassung**

Die Erfindung betrifft eine Therapievorrichtung für Arztpraxen zur Aktivierung der Selbstheilungskräfte eines Patienten, umfassend eine Bestrahlungseinheit (6) mit einer Anzahl Laserdioden für eine lichttherapeutische Behandlung, wobei der Patient nach ärztlicher Anleitung an sich unüberwacht über einen längeren Zeitraum eine auf ihn individuell abstimmbare und veränderbare lichttherapeutische Behandlung kontrolliert vornehmen kann, während der er sich auf sich und bestimmte Körperpartien konzentriert und er hierdurch seine Selbstheilungskräfte aktiviert. Die Erfindung betrifft zudem die Verwendung der Therapievorrichtung, sowie Verfahren zur Aktivierung der Selbstheilungskräfte in Patienten.

## Beschreibung

### TECHNISCHES GEBIET

Die Erfindung betrifft eine Therapievorrichtung für Arztpraxen, insbesondere eine Therapievorrichtung, welche die körpereigenen Selbstheilungskräfte von Patienten aktiviert.

### HINTERGRUND DER ERFINDUNG

Als Selbstheilungskraft bezeichnet man die Fähigkeit des menschlichen Körpers, sowohl äußere als auch innere Verletzungen sowie Krankheiten ohne äußere Einwirkung oder eine medizinische Therapie zu heilen. Nicht nur die Naturheilkunde, sondern auch die moderne Medizin nützt diese Selbstheilungskräfte, und ihnen kommt neben der unmittelbaren therapeutischen Behandlung (Operation, Bestrahlung, Medikamente usw.) eine immer größere Bedeutung zu. Auch gibt es viele Krankheiten, die medizinisch nicht wirklich geheilt werden können. Dazu zählen zum Beispiel neurologische Krankheiten wie Multiple Sklerose, amyotrophe Lateralsklerose, Alzheimer, Parkinson, aber auch chronische Krankheiten wie Diabetes, Rheuma, Schmerzsyndrome, Krebs, AIDS (HIV) usw. Auch akute Erkrankungen, Kapselentzündungen oder langwierige Verletzungen sind oft große therapeutische Probleme, insbesondere, wenn sie mit starken Schmerzen verbunden sind. Sie werden von den Patienten unabhängig von der Schwere der Erkrankung als sehr belastend empfunden und suchen daher das Gespräch beim Arzt in der Hoffnung auf Hilfe. Auch wenn keine wirkliche therapeutische Hilfe angeboten werden kann, so bleibt der Wunsch des Patienten auf Hilfe und Besserung der körperlichen oder zumindest der psychologischen Situation.

Zusammenhänge zwischen der psychischen Situation und dem Verlauf einer Krankheit sind bekannt. Sind Patienten in einer schlechten seelischen Verfassung, kann auch die beste medizinische Versorgung trotzdem nicht helfen. Durch Entspannungs- und Meditationstechniken erhalten Patienten Zugang zu sich selbst und möglichen Selbstheilungskräften, so dass die allgemeine Situation selbst bei unheilbaren oder chronischen Krankheiten als besser empfunden wird und vom Patienten bewältigt werden kann.

A. Olschewski lehrt in Erfahrungsheilkunde 1, 1999, 17-23 (*Selbstheilungskräfte - allgemeine Thesen und praktische Umsetzung am Beispiel der integrativen Arbeit mit Krebskranken*) durch das Hervorrufen spezifischer Phantasien im Patienten die Selbstheilungskräfte zu aktivieren. Diese erfordern eine intensive Therapie, in der der Arzt oder Therapeut mit dem Patienten eine Beziehung herstellen muss, durch die er an seine inneren Ressourcen und Selbstheilungskräfte gelangen kann. Seine Lehre umfasst eine komplizierte Therapie mit etwa zehn verschiedenen Stadien und ist von einem Kassenarzt wegen des Zeitaufwands nicht wirtschaftlich einsetzbar. Zum einen sind die vorgeschlagenen Methoden bei ungewissem Erfolg sehr zeitaufwendig und zum anderen sowohl für Allgemeinmediziner als auch den Spezialisten psychisch sehr belastend.

Der Stand der Technik stellt sich somit als Problem dar.

### KURZBESCHREIBUNG DER ERFINDUNG

Das Problem wird gelöst durch eine Vorrichtung zur Aktivierung der Selbstheilungskräfte eines Patienten, umfassend eine Bestrahlungseinheit mit einer Anzahl Laserdioden für eine lichttherapeutische Behandlung, eine Steuereinheit zur Steuerung der Stärke und der Pulsfrequenzen der Laserdioden in der Bestrahlungseinheit, sowie einen Prozessor zur Darstellung der physikalischen Parameter der Bestrahlung auf einem Monitor. Die Vorrichtung ist weiter dadurch gekennzeichnet, dass der Prozessor ausgelegt ist für eine ärztliche Kommunikation und Interaktion mit dem Patienten und er die vorgesehene Behandlungsform auf dem Monitor durch Schriftund Symbolzeichen sinnfällig für den Patienten darstellt, dass die Bestrahlungseinheit bei der lichttherapeutischen Behandlung vom Patienten von Hand an den Ort der gewünschten therapeutischen Behandlung geführt werden kann; und dass die zur Verfügung stehenden lichttherapeutischen Behandlungsformen für den Patienten im Prinzip unschädlich und ungefährlich sind; so dass der Patient nach ärztlicher Anleitung an sich unüberwacht über einen längeren Zeitraum eine auf ihn individuell abstimmbare und veränderbare lichttherapeutische Behandlung kontrolliert vornehmen kann, während der er sich auf sich und bestimmte Körperpartien konzentriert und er hierdurch seine Selbstheilungskräfte aktiviert.

Die Vorrichtung dient dazu, Patienten, die nicht weiter direkt behandelt werden können - entweder weil sie an einer im medizinischen Sinn unheilbaren Krankheit leiden oder weil sie sich bereits in einer längeren medizinischen Behandlung befinden - eine Bestrahlungstherapie über einen bestimmten Zeitraum anzubieten. Die Bestrahlung mit den Laserdioden ist absolut ungefährlich und bietet dem Patienten eine individuelle und dadurch personenbezogene Behandlung. Der behandelnde Arzt lädt Patientendaten (zum Beispiel Größe, Gewicht, Alter, Geschlecht, Anschrift, Erkrankung, Symptome, usw.) auf die Vorrichtung. Diese Daten können dann während der Behandlung über den Monitor dem Patienten wiedergegeben werden. Dadurch fühlt sich der Patient individuell betreut, was sich positiv auf sein Wohlgefühl und dadurch auf seine allgemeine psychische Verfassung auswirkt. Gleichzeitig braucht der behandelnde Mediziner nur wenige Minuten aufzuwenden, um die Vorrichtung mit Patientendaten zu versorgen und kann sich nach Beginn der Therapiesitzung dann anderen Patienten widmen.

Zudem können der behandelnde Mediziner und der Patient mit der erfindungsgemäßen Vorrichtung Flüssigkeiten zur Einnahme bestrahlen. So können zum Beispiel Wasser oder verdünnte, durchsichtige alkoholische Getränke mit Hilfe der Vorrichtung vom Patienten bestrahlt werden und dann eingenommen werden. Es ist bevorzugt, dass die Flüssigkeit mit derselben Parameterabfolge "therapiert" wird, wie der Patient. Alternativ können auch Feststoffe oder sogar Gase benutzt werden.

Vorteilig an der Vorrichtung ist, dass sie das allgemeine Wohlbefinden des Patienten verbessert und dadurch seine Selbstheilungskräfte aktiviert. Ein weiterer Vorteil liegt darin, dass der Zeitaufwand für den behandelnden Mediziner pro Patient verringert wird.

Zudem hat die Vorrichtung eine geringe Größe und Gewicht und kann somit auch auf Urlaubs- und Geschäftsreisen genutzt werden.

In einer vorteilhaften erfindungsgemäßen Ausführungsform hat die Bestrahlungseinheit der Vorrichtung drei unterschiedliche Laserdioden, vorzugsweise in den Farben rot (639 nm Wellenlänge), grün (532 nm) und blau-violett (405 nm). Die Bestrahlung mit ungefährlichem, schwachem und sichtbarem Laserlicht hat den Vorteil, dass der Patient die Bestrahlungsbehandlung beobachten kann und dass das Therapiegefühl somit weiter gestärkt wird. Die Bestrahlung erfolgt vorzugsweise auf großflächigen Hautpartien, wie auf Brust und Bauch, an den Beinen, den Armen oder auf dem Rücken. Der Patient kann selbständig entscheiden, auf welche Hautpartien er die Behandlung anwendet; der behandelnde Mediziner kann dabei eine beratende Funktion übernehmen.

In einer bevorzugten Ausführungsform können die Laserdioden unabhängig voneinander mit beliebigen Pulsfrequenzen strahlen, wie zum Beispiel Pulsfrequenzen zwischen 0,1 Hz und 1000 GHz, oder Pulsfrequenzen zwischen 1 Hz und 1000 GHz oder Pulsfrequenzen zwischen 1 Hz und 1 GHz oder Pulsfrequenzen zwischen 1 Hz und 20 MHz.

In einer weiteren Ausführungsform verfügt die Vorrichtung zudem über ein Lesegerät für ein Speichermedium, zum Beispiel für eine SD-Speicherkarte. Dies ermöglicht es dem behandelnden Mediziner, Patientendaten auf dem Speichermedium zu speichern und die Vorrichtung schneller mit Patientendaten zu versorgen. Wahlweise kann der Patient selbständig die Vorrichtung mit den Daten versorgen, so dass eine weitere Zeitersparnis für den behandelnden Mediziner entsteht.

Es ist weiterhin möglich, dass der Patient über die Steuerung die Behandlungsmethode selber in einem begrenzten Umfang beeinflusst. So kann er die Dauer, die Intensität, das bevorzugte Laserlicht (rot, grün oder blau-violett), die Pulsfrequenz oder die Bestrahlungsfläche selber festlegen. Dadurch, dass dem Patienten diese Möglichkeit gegeben wird, wird der Eindruck verstärkt, dass er seine Therapie selber steuern kann, ohne aber vom behandelnden Mediziner vernachlässigt zu werden, was sein Selbstwertgefühl und Eigeninitiative verstärkt. Dies führt zu einer weiteren Verbesserung der psychischen Verfassung und kann die Selbstheilungskräfte weiter aktivieren.

Der Prozessor vermittelt dem Patienten über den Monitor persönliche Daten, wie Name, Alter, Größe, Gewicht, Geschlecht, Erkrankung, Symptome usw. sowie die Behandlungsmethode (mit Lichtintensitäten der einzelnen Laserdioden, Bestrahlungsdauer und Pulsfrequenz, Therapiedauer und -stadium). Zudem kann der Monitor ein berührungsempfindlicher Monitor (Touchscreen) sein, über den der Patient der Vorrichtung Befehle erteilt, um die Therapie selbständig zu gestalten. Über einen berührungsempfindlichen Monitor kann der Patient in einer Ausführungsform der Erfindung die Steuereinheit anweisen, bestimmte Therapieparameter, wie Dauer, Intensität, bevorzugte Laserdioden, Pulsfrequenz usw. zu verändern. Es ist dabei möglich, die Parameter so festzulegen, dass die einzelnen Laserdioden mit unterschiedlichen Intensitäten und Pulsfrequenzen leuchten.

Ebenfalls Teil der Erfindung ist die Verwendung der Vorrichtung zur Aktivierung der Selbstheilungskräfte eines Patienten in der Behandlung. In einer Ausführungsform bezieht sich die Verwendung spezifisch auf die Schmerzbehandlung. Die erfindungsgemäße Vorrichtung kann ebenso zur Behandlung von chronischen sowie akuten Erkrankungen verwendet werden, wie zum Beispiel amyotrophe Lateralsklerose, Multiple Sklerose, Parkinson, Alzheimer, Gelenkkrankheiten, Diabetes, Rheuma, Krebs, AIDS (HIV), Kapselentzündungen usw., oder bei Verletzungen wie Knochenbrüche, Verbrennungen oder Muskelverletzungen.

Bei der Verwendung der erfindungsgemäßen Vorrichtung werden mit der Bestrahlungseinheit bevorzugt Hautpartien des Patienten bestrahlt. Zudem kann eine Anzahl nicht-toxische Feststoffe, Flüssigkeiten oder Gase bestrahlt werden, die der Patient als Teil der Therapie zu sich nimmt, beziehungsweise inhaliert. In einer bevorzugten Verwendungsform erfolgt die Bestrahlung der Hautpartien und der Anzahl nicht-toxische Feststoffe, Flüssigkeiten oder Gase nach dem gleichen Muster.

Des Weiteren ist Teil der Erfindung ein Verfahren zur Aktivierung der Selbstheilungskräfte eines Patienten, umfassend die Bestrahlung von Hautpartien der Patienten mit elektromagnetischer Strahlung, und/oder die Bestrahlung von Feststoffen, Flüssigkeiten und/oder Gasen zur Verabreichung an den Patienten. Es ist bevorzugt, dass die Bestrahlung der Hautpartien und der Feststoffe, Flüssigkeiten oder Gase nach dem selben Muster erfolgt. In einer alternativen Ausführungsform des Verfahrens sind die zu verabreichenden Stoffe mit elektromagnetischer Strahlung von verschiedenen Wellenlängen bestrahlt worden. Die Verabreichung kann in der Form von Schlucken, Inhalation, Injektion, Infusion oder in jeder anderen Form erfolgen.

Zudem ist Teil der Erfindung ein Verfahren zur Herstellung von aktivierten Stoffen für die Aktivierung der Selbstheilungskräfte eines Patienten, umfassend das Bereitstellen eines Stoffes und dessen Bestrahlung mit elektromagnetischer Strahlung. Die Bestrahlung kann durch jedwede Form elektromagnetischer Strahlung erfolgen, deren Quelle ein Frequenzgenerator sein kann, wie Laser, magnetische Spulen, Zapper mit rechteckigen Wellen, Tens-Geräte mit sinusförmigen Wellen oder Skalarwellen, Elektroakupunkturvorrichtungen, Biophotonenstrahler, Lakhovsky-Spiralen, Tachyonenquellen oder beliebige andere Quellen. In einer alternativen Ausführungsform ist die Übertragung der Aktivierung von einem aktivierten Stoff auf einen nicht-aktivierten Stoff mit Hilfe von Orgonstrahlern oder Intentionen möglich.

Weiter Teil der Erfindung sind aktivierte Stoffe für die Aktivierung der Selbstheilungskräfte bei Patienten die nach den oben beschriebenen Verfahren hergestellt sind.

Die Erfindung wird nun in weiteren Einzelheiten an Hand von Zeichnungen und Beispielen beschrieben.

### KURZBESCHREIBUNG DER ZEICHNUNGEN

Es zeigt:
- Fig. 1: eine Ausführungsform der erfindungsgemäßen Vorrichtung zur Aktivie- rung der Selbstheilungskräfte eines Patienten mit Steuerung, Bestrah- lungseinheit und Schnittstelle;
- Fig. 2: eine Vorderansicht der erfindungsgemäßen Vorrichtung im Bereit- schaftsbetrieb;
- Fig. 3: eine Rückansicht der Steuerung;
- Fig. 4, 5: eine Ansicht des Bildschirms der erfindungsgemäßen Vorrichtung mit Fehlerwarnung;
- Fig. 6: eine Ansicht des Bildschirms der erfindungsgemäßen Vorrichtung mit Statusangaben;
- Fig. 7: eine Ansicht des Bildschirms der erfindungsgemäßen Vorrichtung mit Anwendermenü zur Auswahl von Benutzereinstellungen;
- Fig. 8 bis 10: eine Ansicht des Bildschirms der erfindungsgemäßen Vorrichtung mit Anwendermenüs für die Auswahl von Sprach-, Ton- und Helligkeitsein- stellungen;
- Fig. 11: eine Ansicht des Bildschirms der erfindungsgemäßen Vorrichtung mit Anwendermenü zur Auswahl von Therapieparametern;
- Fig. 12: eine Ansicht des Bildschirms der erfindungsgemäßen Vorrichtung mit Anwendermenü zur Einstellung der Therapiedauer;
- Fig. 13: eine Ansicht des Bildschirms der erfindungsgemäßen Vorrichtung mit Anwendermenü zur Einstellung der Pulsfrequenz;
- Fig. 14: eine Ansicht des Bildschirms der erfindungsgemäßen Vorrichtung mit Anwendermenü zur Einstellung der Therapieverzögerung;
- Fig. 15 bis 18: Ansichten des Bildschirms der erfindungsgemäßen Vorrichtung mit An- gabe von Therapiedaten;
- Fig. 19: eine Ansicht des Bildschirms der erfindungsgemäßen Vorrichtung mit Angabe con Patientendaten;
- Fig. 20: eine Ansicht des Bildschirms der erfindungsgemäßen Vorrichtung mit Anwendermenü zur Auswahl des Patienten;
- Fig. 21, 22: beispielhafte Ausführungsformen der Steuereinheit in der erfindungsge- mäßen Vorrichtung.

### EINGEHENDE BESCHREIBUNG DER ERFINDUNG

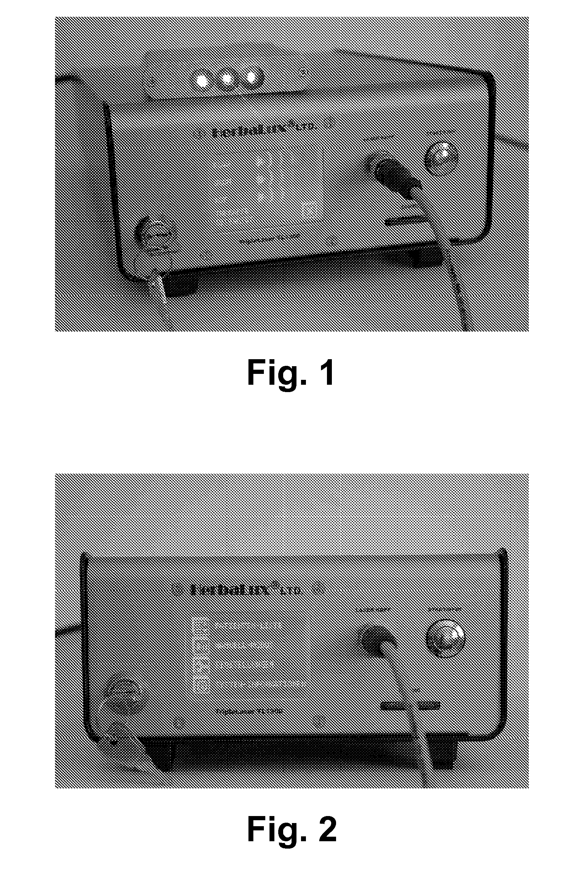

Fig. 1 zeigt eine Ausführungsform der Erfindung in betriebsbereitem Zustand. Die erfindungsgemäße Vorrichtung zur Aktivierung der Selbstheilungskräfte eines Patienten umfasst einen Monitor 2 für die Interaktion zwischen Vorrichtung und Patient, eine Steuereinheit 4, eine Bestrahlungseinheit 6 mit Leuchtdioden 6a, 6b, 6c (in rot, grün und blau-violett) und ein Datenkabel 8 zur Datenübertragung zwischen Steuereinheit 4 und Bestrahlungseinheit 6. Zudem sind zu sehen ein Lesegerät 10, ein Schlüsselschalter 12 zum Ein- und Ausschalten der Vorrichtung und ein Drucktaster 14 mit Start-Stopp-Funktion zum Beginnen, Unterbrechen und Beenden der Therapie.

In der gezeigten beispielhaften Ausführungsform der Erfindung kann der Patient selbständig wählen, wo er die Bestrahlung anwendet. Er tut dies, indem er die Bestrahlungseinheit 6 auf eine beliebige Hautpartie richtet, zum Beispiel eine Hautpartie an seinem Unterarm, Oberarm, Bein oder an anderen Körperstellen. Beispielsweise kann der Patient mit der Bestrahlungseinheit 6 Melanome eines Hautkrebses bestrahlen.

Über das Lesegerät 10 kann der behandelnde Mediziner der Vorrichtung persönliche Daten des Patienten übermitteln, wie Name, Alter, Größe, Gewicht, Geschlecht, Erkrankung, Symptome usw. In der gezeigten beispielhaften Ausführungsform ist das Lesegerät 10 ein Lesegerät für eine SD-Speicherkarte. Andere Lesegeräte, wie Lesegeräte für Floppy-Disks, USB-Speicher, CD-ROMs, DVDs oder andere Speichermedien können auch benutzt werden. Vorteilhaft ist dabei die Geschwindigkeit der Datenübertragung an die Vorrichtung. Der behandelnde Mediziner kann die Daten von verschiedenen Patienten auf verschiedenen SD-Speicherkarten aufbewahren und diese dann nach Bedarf, vor der Therapiesitzung eines bestimmten Patienten, auf die Vorrichtung aufladen. Es ist auch denkbar, dass der Patient selber seine persönlichen Daten auf die Vorrichtung auflädt. Vorteilhaft daran ist die erhebliche Zeitersparnis, die damit für den behandelnden Mediziner entsteht. So muss der behandelnde Mediziner nur wenige Minuten aufwenden, um die Vorrichtung für die Sitzung eines bestimmten Patienten zu konfigurieren, die eine beliebig lange Zeit, zum Beispiel eine halbe oder eine ganze Stunde, dauern kann.

Bei Einschalten der Vorrichtung befindet sie sich normalerweise im Zustand wie in Fig. 2 gezeigt. Die Bestrahlungseinheit (nicht zu sehen) ist über das Datenkabel 8 mit der Vorrichtung verbunden und kann vom Patienten in eine beliebige Position bewegt werden. Auf dem Bildschirm 2 erscheint ein Menü, über das der behandelnde Mediziner bzw. der Patient selber Einstellungen vornehmen oder die Therapiesitzung beginnen kann.

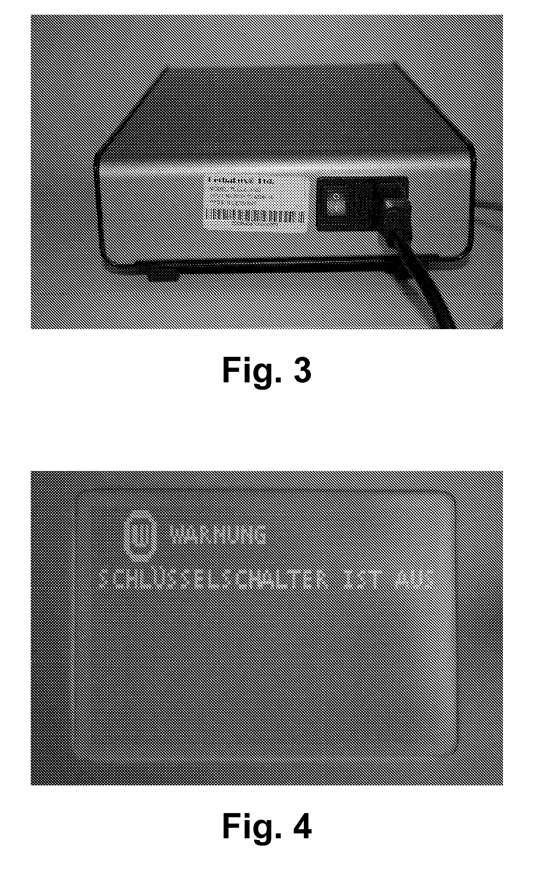

Fig. 3 zeigt eine Rückansicht der Steuerung 2 der Vorrichtung, mit einem Netzkabel 16 zur Stromversorgung und einem Hauptschalter 18 zum Ein- und Ausschalten der gesamten Vorrichtung.

Es werden jetzt verschiedene Stadien und Einzelheiten bei der Anwendung der erfindungsgemäßen Vorrichtung beschrieben. Fig. 4 zeigt eine Fehlermeldung, die angibt, dass die Vorrichtung mit dem Schlüsselschalter 12 abgeschaltet ist. Zum Einschalten muss der Schlüsselschalter 12 betätigt werden. Dies ermöglicht es dem Besitzer der Vorrichtung, zum Beispiel dem behandelnden Mediziner, Unbefugten die Benutzung der Vorrichtung zu verbieten und so etwaigen Missbrauch zu unterbinden.

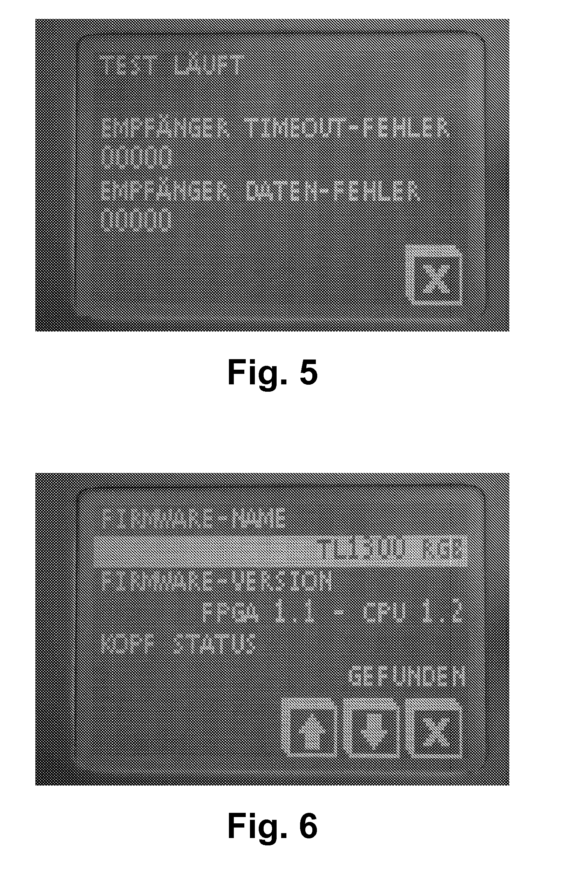

Fig. 5 zeigt eine weitere Fehlermeldung, die angezeigt wird, wenn keine Verbindung zwischen der Steuerung 2 und der Bestrahlungseinheit 6 hergestellt werden kann. In dem Fall sollte das Datenkabel 8 überprüft werden. Wird der Bildschirm wie in Fig. 6 mit Statusdaten der Vorrichtung angezeigt, so ist sie betriebsbereit und die Therapiesitzung kann beginnen.

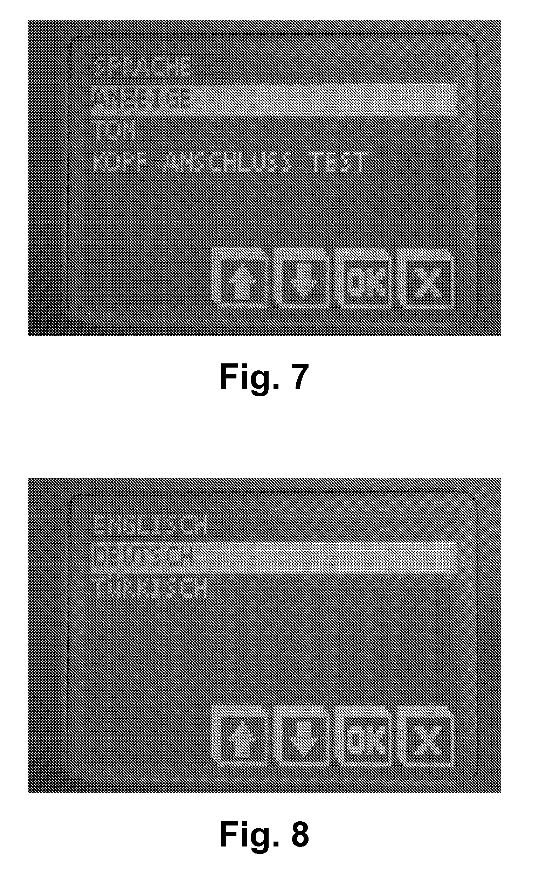

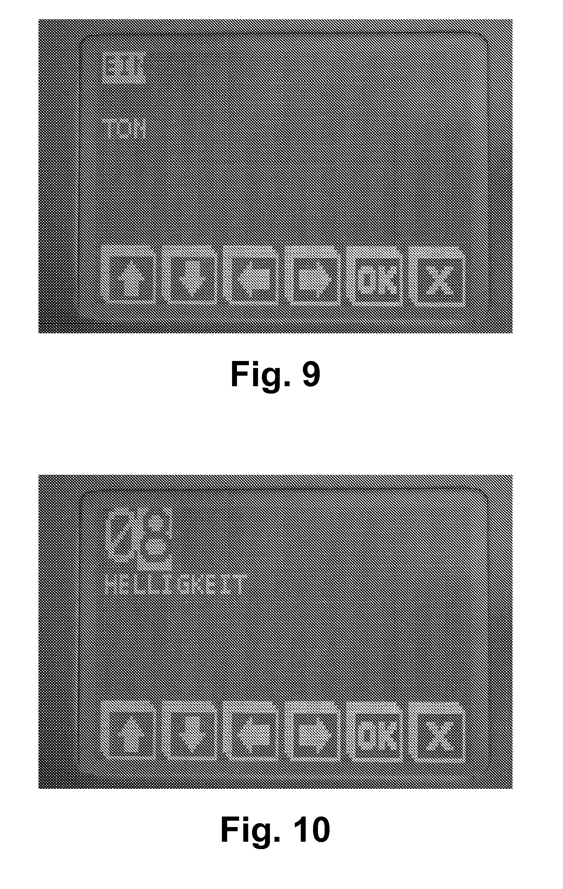

Durch Wahl der Menü-Option "Einstellungen" kann der Bildschirm aus Fig. 7 angezeigt werden, mit dem der behandelnde Mediziner oder der Patient die Sprache (mehrere Sprachen zur Auswahl, je nach Ausführungsform), die Anzeige (Helligkeit, Kontrast) und den Ton (Lautstärke, Ein/Aus) der Vorrichtung bestimmen kann. Fig. 8 bis 10 zeigen verschiedene beispielhafte Einstellungsmöglichkeiten.

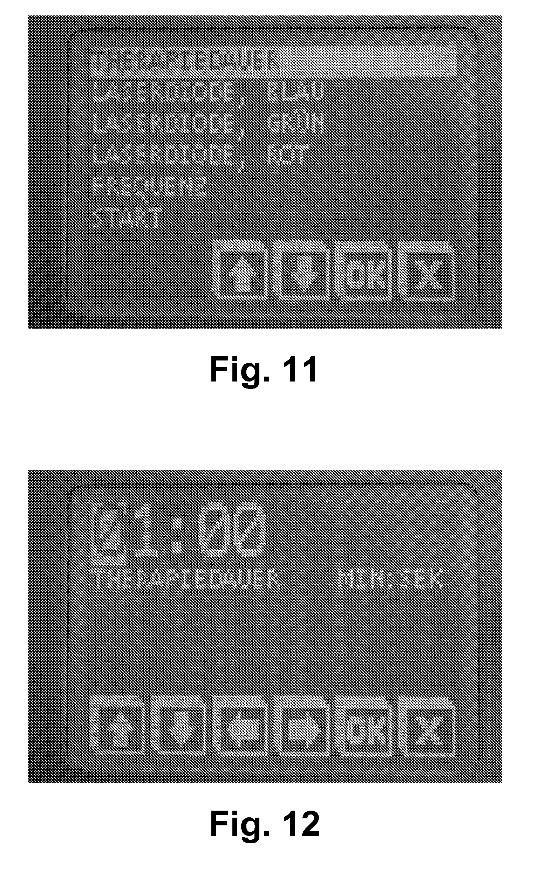

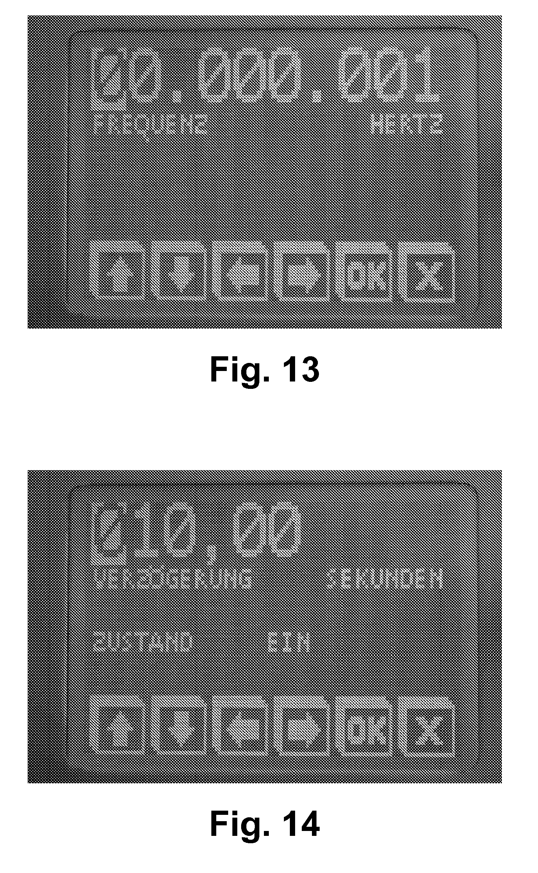

Über den Bildschirm aus Fig. 11 können Einstellungen der Therapie vorgenommen werden. Dabei können, wie gezeigt, die Dauer der Therapie (Fig. 12), die Pulsfrequenz (Fig. 13), sowie die Intensität der einzelnen Laserdioden 6a, 6b, 6c eingestellt werden. Zudem ist es möglich, mit der Funktion aus Fig. 14 eine Verzögerung der Funktion von einer oder mehreren Leuchtdioden in die Sitzung einzubauen.

Die Einstellungen ergeben sich entweder aus den persönlichen Patientendaten, die der Vorrichtung zugeführt worden sind, oder sie werden manuell eingestellt. Die genaue Therapie obliegt dabei der Verantwortung des behandelnden Mediziners, wobei der behandelnde Mediziner auch dem Patienten eine gewisse Eigenverantwortung beim Bestimmen der Therapie, gegebenenfalls innerhalb bestimmter Grenzen, überlassen kann.

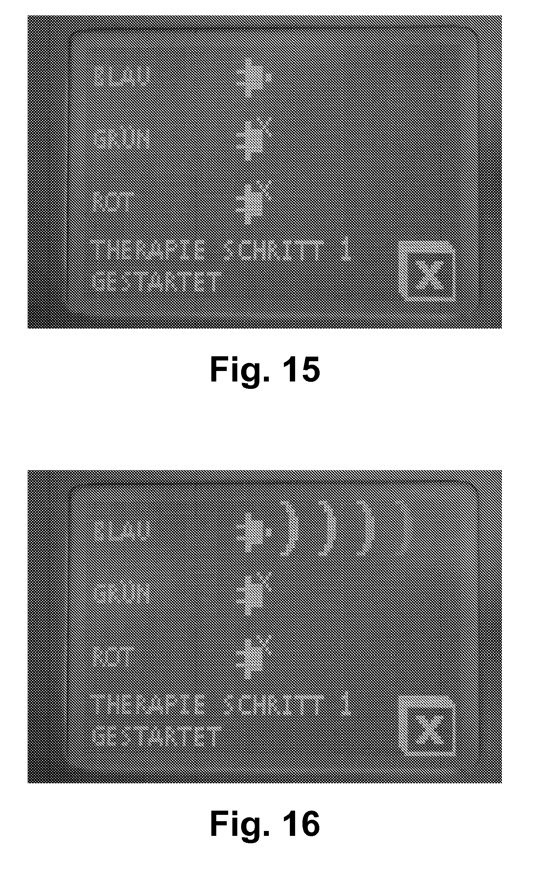

Wird die Sitzung begonnen, so erscheinen auf dem Monitor Therapieangaben, wie in den Fig. 15 und 16 gezeigt. Damit wird dem Patienten gezeigt, in welchem Therapieschritt (hier: Therapieschritt "1") er sich befindet, und welche der Laserdioden bei dem Therapieschritt aktiviert beziehungsweise deaktiviert sind. Zudem können mit Hilfe geeigneter Symbole, Blinken oder Bewegungen auf dem Monitor, oder auch durch Tonsignale, Pulsfrequenz und Intensität der Bestrahlung durch die einzelnen Laserdioden angezeigt werden.

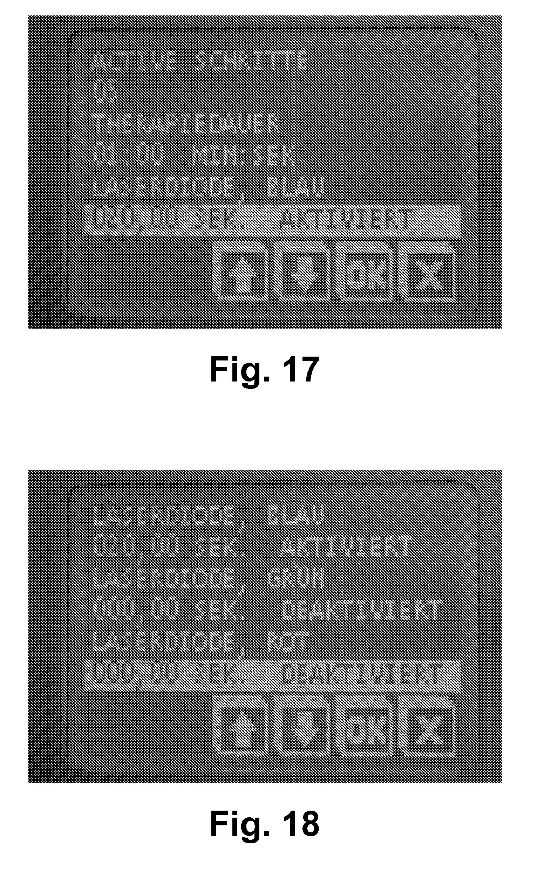

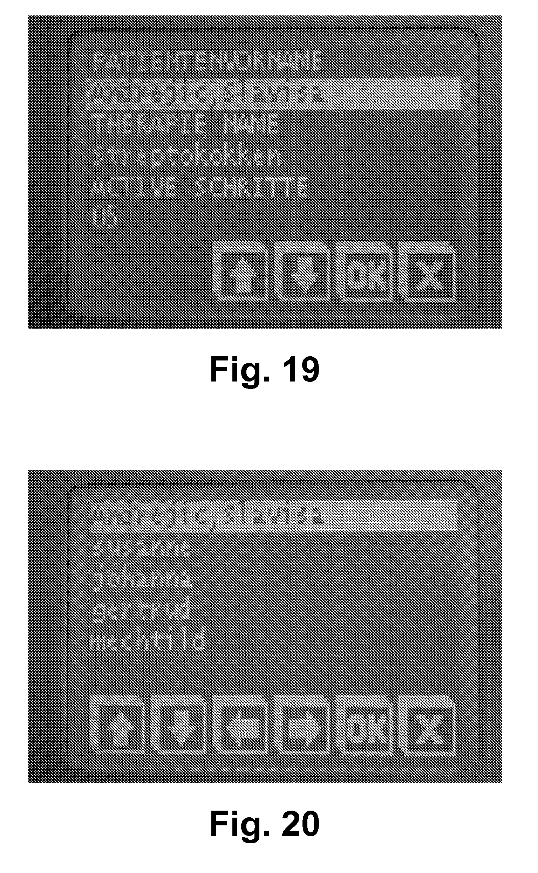

Mit den Bildschirmen, die in Fig. 17 bis 20 gezeigt sind, erhält der Patient weitergehende Informationen über die Therapie. Insbesondere durch Angabe des Namens des Patienten wird eine individuell angepasste persönliche Therapie des Patienten nahegelegt.

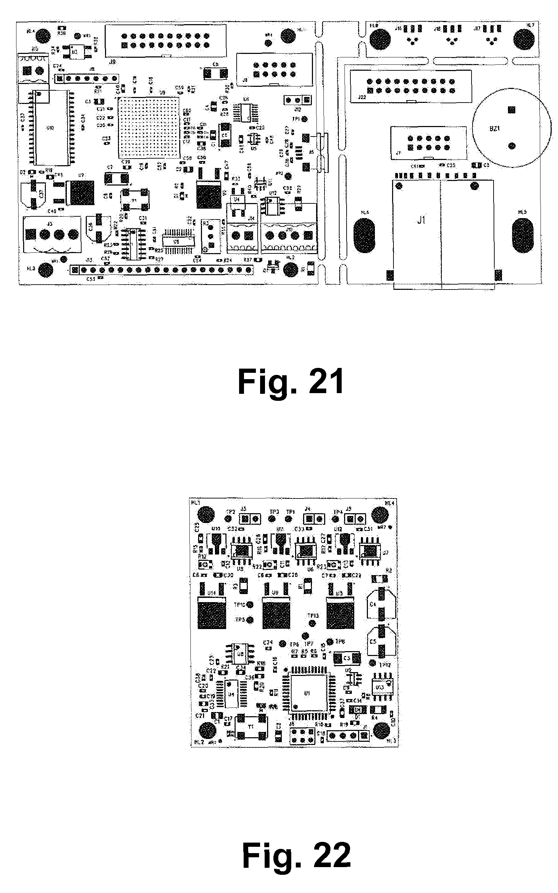

Beispielhafte Ausführungsformen der Steuereinheit 4 als Steuerplatine sind in den Fig. 21 und 22 angegeben.

Für den Patienten entstehen durch die Therapie zweierlei Vorteile. Einerseits kann er beliebige Hautstellen therapieren, indem er die Bestrahlungseinheit 6 auf die betreffenden Hautstellen richtet. Dadurch, dass die Bestrahlungseinheit Laserlicht im sichtbaren Bereich aussendet, ist für den Patienten klar ersichtlich, worin die Therapie praktisch besteht und wie die betroffenen Hautstellen bestrahlt werden. Zudem kann der Patient bei nicht zufriedenstellender Wirkung der Bestrahlung entweder selbständig, oder mit Hilfe des behandelnden Mediziners, eine Änderung der Einstellungen vornehmen. Dadurch kann der Patient Einfluss auf seine Therapie nehmen und bekommt nicht das Gefühl, dass er dem behandelnden Mediziner ausgeliefert ist.

Andererseits bietet sich dem behandelnden Mediziner die Möglichkeit, eine Therapie anzubieten, die ohne viel Zeitaufwand beim Patienten sichtbare Auswirkungen hat und eine auf ihn abgestimmte individuelle Behandlung nahelegt, so dass sich dieser ernst genommen und verstanden fühlt, mit den sich daraus ergebenden positiven Auswirkungen auf den psychischen Zustand des Patienten. Für den behandelnden Mediziner eröffnet dies die Möglichkeit, sich Patienten anzunehmen, die nicht oder nur schwer therapierbar sind, und dies bei geringem eigenen Zeitaufwand. So muss der behandelnde Mediziner für eine 30-minütige Sitzung für den Patienten im Durchschnitt - mit Ausnahme der ersten Sitzung, bei der eine Einweisung notwendig ist - nicht mehr als 3 bis 5 Minuten persönlich aufwenden.

Die erfindungsgemäße Vorrichtung bietet also erhebliche Vorteile, sowohl für den Patienten, als auch für den behandelnden Mediziner.

Bei der Verwendung der Vorrichtung durch einen Patienten, gegebenenfalls unter Aufsicht des behandelnden Arztes können zudem bestrahlte Stoffe für die Aktivierung der Selbstheilungskräfte des Patienten zum Einsatz kommen. Durch vorherige Bestrahlung sind diese Stoffe aktiviert und verstärken so den Effekt der Strahlungsbehandlung zur Aktivierung der Selbstheilungskräfte. Es ist dabei vorteilhaft, wenn die bestrahlten Stoffe nach dem selben Muster, das heißt mit der selben Wellenlänge, Pulsfrequenz, Intensität und Dauer bestrahlt werden, als die Hautpartien des Patienten. Es hat sich herausgestellt, dass wenn ein Patient gleichzeitig mit einer Strahlentherapie auch noch bestrahltes Wasser, bevorzugt in kleinen Schlucken, zu sich nimmt, der Effekt der Therapie noch verstärkt wird. Dieser synergetische Effekt kann noch weiter erhöht werden, indem das Wasser nach demselben Muster bestrahlt wird als die Haut des Patienten.

Es können auch andere Substanzen zum Einsatz kommen als Wasser, nämlich alkoholische und nicht-alkoholische Flüssigkeiten, aber auch Feststoffe, wie Lebensmittel, Globuli oder Tabletten zum Einnehmen und sogar Gase zum Inhalieren. Es können sogar physiologische Kochsalzlösungen genutzt werden, die dem Patienten während der Bestrahlung injiziert werden. Auch ist es möglich, weitere bestrahlte Objekte, wie zum Beispiel Steine, Kristalle, Glas, Plexiglas, Wachs oder Pflanzen oder Pflanzenteile zu nutzen, die während der Therapie in dem Raum abgestellt werden, in dem sich der Patient befindet. Je nach Therapie kann der behandelnde Arzt entscheiden, welche bestrahlte Substanz oder Substanzen zum Einsatz kommen sollen.

Das Verfahren zum Aktivieren der Stoffe, die mit der erfindungsgemäßen Vorrichtung zum Einsatz kommen, ist auch Teil der Erfindung. Die Stoffe können mit elektromagnetischer Strahlung aktiviert werden, die aus einer beliebeigen Quelle stammen kann. Die Strahlung kann verschiedene Formen haben, es können also Skalarwellen, rechteckige Wellen, sinusförmige Wellen oder andere unregelmäßig geformte Wellen zum Einsatz kommen. Als Strahlungsquellen kommen daher in Frage sichtbare, UV- und IR-Laser, magnetische und elektromagnetische Spulen, Elektroakupunkturvorrichtungen, Tens-Geräte, Zapper, Biophotonenstrahler, handelsübliche Kakhovsky-Spiralen ("Mehrwellen-Oszillator", beschrieben im US-Patent 1,962,565), oder Tachyonenquellen. Zudem kann die Aktivierung eines aktivierten Stoffes auch auf einen nicht aktivierten Stoff übertragen werden, und zwar mit Hilfe von Orgon-Strahlern oder Intentionen.

Stoffe, die mit dem oben genannten Verfahren aktiviert worden sind und bei der Aktivierung der Selbstheilungskräfte in Patenten zum Einsatz kommen können sind ebenfalls Teil der Erfindung.

Die Schmerzmessung in den folgenden Beispielen erfolgte über eine Eigeneinschätzung der Patienten. Dabei wurden die Patienten regelmäßig befragt, als wie stark sie ihre Schmerzen einschätzen, und dies auf einer Skala zwischen 0 (kein Schmerz) und 10 (höchste Schmerzstufe) einzutragen. Eine Schmerzverringerung um einen bestimmten Prozentsatz entspricht demnach einer Verringerung der Werte aus der Eigeneinschätzung des Patienten auf dieser Skala.

### BEISPIEL 1

Eine beispielhafte Vorrichtung zur Aktivierung der Selbstheilungskräfte eines Patienten wird wie folgt beschrieben:

Die Vorrichtung zur Aktivierung der Selbstheilungskräfte eines Patienten besteht aus einem Bedienterminal, einem Laserkopf und Benutzer-Software für Microsoft Windows. Sie ist ein medizinisches Gerät, das Laserstrahlen in den Farben rot, grün und blau-violett generiert. Die Pulsfrequenzen der Laserstrahlen sind einstellbar zwischen 1 Hertz und 20 Megahertz.

Der Anwender (behandelnder Mediziner, Arzt, Facharzt oder Naturheilkundler) kann mit der PC-Software folgende Patientendaten auf eine SD-Karte schreiben und abrufen:
- Name, Vorname, Anschrift, Alter, Gewicht usw.;
- Beschreibung der Therapie;
- Frequenz der Therapie;
- Einschaltverzögerung zwischen den Laserdioden;
- Behandlungszeit;
- Wahl zwischen den drei Laserdioden.

Das Gerät ist mit einem berührungsempfindlichen Monitor ausgestattet. Der Anwender kann auf dem Bildschirm die jeweilige Therapie aus einer Liste auswählen. Möglich ist es auch, die Einstellungen der Therapie manuell einzugeben. Durch Betätigen der Starttaste kann der Anwender die Therapie starten und eventuell stoppen. Nach erfolgter Behandlungszeit stoppt das Gerät automatisch und erzeugt ein akustisches Signal.

Das Bedienterminal kann die Daten auf der SD-Karte nicht ändern. Diese können nur mit der PC-Software geändert werden. Das Bedienterminal umfasst die folgenden Bauteile:
- Gehäuse aus Aluminium, Farbe Gold, Profil ca. 1,5 bis 2 mm, mit schwarzer Beschriftung;
- Rückseite, mit Etikette versehen;
- 128 × 64 Grafik-LCD mit Touch Panel, LED-Beleuchtung Weiß-Blau;
- Schlüsselschalter, 2 Positionen (ein/aus), Farbe Silber;
- Drucktaste, Start-Stopp-Funktion, Farbe Silber;
- Anschluss für SD Karte, Minimum 512 KB SD Karte;
- Kabelstecker für den Laserkopf;
- Steckdosenmodul mit Filter, Sicherung, Ein-Aus Schalter;
- Netzkabel, minimal 1 Meter;
- Internes Netzteil, universal, 85-264 VAC, 50/60 Hz; und
- Interne Steuerplatine mit FPGA und integriertem Mikroprozessor.

Der Laserkopf umfasst:
- Fertiges Gehäuse, Farbe Gold, Profil ca. 1,5 bis 2 mm;
- Kabelstecker und Etikette an der Rückplatte, ca. 3 bis 5 mm Öffnung an der Frontplatte für Laserstrahlen;
- Laserdiode, rot, 639 nm Wellenlänge, maximal 12 mW Leistung;
- Laserdiode, grün, 532 nm Wellenlänge, maximal 5 mW Leistung;
- Laserdiode, blau-violett, 405 nm Wellenlänge, maximal 12 mW Leistung;
- drei Glaslinsen;
- innere mechanische Konstruktion um die Laserdioden und die Linse zu befestigen; der Abstand zwischen der Laserdiode und der Glaslinse ist einstellbar; um die Glaslinse einstellen zu können, muss man die Gehäuse öffnen (nur für rot und grün);
- Platine mit 1 Hertz bis 20 Megahertz Signalgenerator; und
- Datenkabel, geschirmt, 1 bis 5 Meter.

Die angewandte PC-Software unterstützt Microsoft Windows 2000/XP/Vista und arbeitet mit einer Standard Windows Oberfläche, in der Sprache Englisch.

### BEISPIEL 2

Die Anwendung der Vorrichtung aus Beispiel 1 bei der Behandlung eines Patienten wird hier beschrieben.

Bei einem 47jährigen Mann mit starker Arthrose in den Kniegelenken konnten trotz intensiver Behandlung mit Steroiden und individuellen Eigenübungen über einen Zeitraum von 3 Monaten nur leichte Fortschritte bei der Behandlung festgestellt werden. Der Patient litt unter starken Schmerzen und hatte große Schwierigkeiten, sich zu bewegen. Da alle belegte Behandlungsmethoden ausgeschöpft waren, konnte die klassische Medizin keine weitere spezifische Therapie anbieten. Der Patient litt zunehmend unter Niedergeschlagenheit und Mutlosigkeit und sein Zustand begann sich zu verschlechtern.

Mit Hilfe der Vorrichtung aus Beispiel 1 wurde dem Patienten ermöglicht, seine Selbstheilungskräfte zu aktivieren. Dazu hat er einem Arzt seinen gesamten Krankheitsverlauf mit Symptomen, Behandlungsversuchen und -erfolgen bzw. -misserfolgen geschildert. Diese Daten wurden, zusammen mit persönlichen Daten des Patienten, auf eine SD-Speicherkarte gespeichert und an die Vorrichtung für die Aktivierung der Selbstheilungskräfte aus Beispiel 1 übertragen.

Dem Patienten wurden anfänglich zehn 30-minütige Therapiesitzungen mit der erfindungsgemäßen Vorrichtung vorgeschlagen. Bei der ersten Sitzung wurde der Patient nach kurzer Einweisung durch den Arzt allein gelassen. Dabei wurde das Therapieprogramm mit Dauer, Auswahl der Leuchtdioden, Pulsfrequenz und Intensität vom Arzt programmiert und über die Steuereinheit kontrolliert. Dem Patienten war es lediglich freigestellt, die Hautpartien auszuwählen, auf die die Bestrahlung gerichtet sein sollte, vorzugsweise Hautpartien auf oder nahe den entzündeten Kniegelenken. Nach der ersten Sitzung wurde ein Kurzgespräch zwischen Arzt und Patient geführt, um den Effekt der Therapie auf dessen Wohlbefinden zu beurteilen. Für die zweite Therapiesitzung wurde das Therapieprogramm auf Basis des stattgefundenen Gesprächs angepasst. Zudem wurde dem Patienten ermöglicht, die Therapie zu beeinflussen, indem ihm in bestimmten Grenzen die Auswahl der Leuchtdioden, deren Pulsfrequenz und Intensität anvertraut wurde. Bei jeder Therapiesitzung wurde dem Patienten mehr Entscheidungsfreiheit eingeräumt und nach jeder Sitzung gab es ein Kurzgespräch zwischen Arzt und Patient.

Bei jeder Therapiesitzung wurde dem Patienten auch ein Glas Leitungswasser zur Verfügung gestellt, das er mit Hilfe der Vorrichtung aus Beispiel 1 beliebig bestrahlen konnte. Der Patient wurde des Weiteren angewiesen, das Wasser in bevorzugt kleinen Schlucken während der Sitzung zu trinken.

Schon nach der zweiten Therapiesitzung konnte beim Patienten eine deutliche Verbesserung der psychologischen Verfassung festgestellt werden. Dadurch, dass der Patient sich durch den Arzt gekümmert und ernst genommen fühlte, und dadurch, dass ihm das Gefühl vermittelt wurde, aktiv etwas für die Verbesserung seines Gesundheitszustandes zu tun, fasste er neuen Mut und neue Zuversicht bezüglich der existierenden Heilungsaussichten. Gleichzeitig gab der Patient an, dass die Schmerzen merklich nachließen.

Über die gesamte Dauer von zehn 30-minütigen Therapiesitzungen konnte so eine deutliche und nachhaltige Verbesserung des psychologischen und physischen Gesundheitszustands des Patienten erreicht werden.

### BEISPIEL 3

Ein 65jähriger Arzt erlitt einen Tauchunfall, der zu einem Th-9-Syndrom (inkomplette Querschnittslähmung) führte. Er konnte sich nur mit Gehstock fortbewegen und litt dabei unter extrem starken Schmerzen, die sich mit herkömmlichen Schmerzmitteln nur ungenügend lindern ließen. Um mobil zu bleiben, suchte er nach einer Alternativmöglichkeit zur Schmerzlinderung. Etwa 30 Jahre nach dem Unfall begann er, sich mit Hilfe der Vorrichtung aus Beispiel 1 selbst zu behandeln, was durch die auf der Speicherkarte dargestellte Diagnose und Patientendaten ermöglicht wurde. Mit Hilfe der Daten auf der Speicherkarte wurde es dem Arzt ermöglicht, sich selber zu therapieren, und zwar mit einer individuell auf ihn abgestimmten Therapie zur Aktivierung seiner Selbstheilungskräfte.

Anfänglich therapierte er sich selbst 2 mal täglich je 10 Minuten. Nach etwa 2 Monaten war nur noch eine tägliche Therapie von 10 Minuten erforderlich. Eine Schmerzreduzierung von bis zu 80% konnte erzielt werden. Durch die Reduzierung der Schmerzen ist auch der Gang des Arztes flüssiger geworden und seine allgemeine Lebensqualität hat sich merklich verbessert.

### BEISPIEL 4

Ein 15jähriger Patient litt unter einer Kniegelenkentzündung mit Gelenkerguss (juvenile idiopathische Oligoarthritis). Er konnte trotz medikamentöser Therapie nur unter Schmerzen gehen und war deswegen vom Schulsport befreit.

Durch eine sorgfältige individuelle Auswahl der Pulsfrequenz der Laserdioden unter Einbeziehung der anamnestischen Daten gelang es nach 10 Sitzungen ä 30 Minuten Schmerzfreiheit zu erzielen. Die Dauer zwischen den Sitzungen betrug jeweils eine Woche. Dem Patienten wurde so ermöglicht, sich wieder am Schulsport zu beteiligen.

### BEISPIEL 5

Eine 29jährige Borreliose-Patientin litt unter extremen Kopfschmerzen, die bereits zu einer 10monatigen Arbeitsunfähigkeit geführt hatten. Eine Therapie mit dem Gerät aus Beispiel 1 bei einer individuell angepassten Zusammenstellung der Laserfrequenzen führte nach 7 Sitzungen, die täglich durchgeführt wurden, zu einer Reduzierung der Schmerzen um etwa 50%. Nach einer 4wöchigen Pause wurden 10 weitere Therapiesitzungen im Abstand von jeweils 2 Tagen durchgeführt, wodurch eine weitere Schmerzlinderung von 10% erzielt wurde. Die Patientin konnte sich daraufhin wieder ins Berufsleben integrieren.

### BEISPIEL 6

Eine Patientin mit Neuroborreliose litt an Schmerzen des gesamten Skelettsystems, unter anderem der linken Hüfte und der Lendenwirbelsäule. Eine antibiotische Therapie war ohne Erfolg verlaufen. Nach 20 Therapiesitzungen innerhalb von 10 Wochen bei einer jeweiligen Sitzungsdauer von 30 Minuten wurde eine Schmerzlinderung von etwa 70% erzielt. Während der Behandlungen wurde der Laser ausschließlich an den schmerzenden Stellen aufgesetzt, nämlich im laufenden Wechsel an den Armen, der Hüfte, der Wirbelsäule, der Füße und der Hände. Durch die Schmerzlinderung wurde sowohl das allgemeine Krankheitsbild der Patientin verbessert, als auch ihre Lebensqualität merklich zum Positiven beeinflusst.

### BEZUGSZEICHENLISTE

- 2: Schnittstelle
- 4: Steuerung
- 6: Bestrahlungseinheit
- 6a, 6b, 6c: Laserdioden
- 8: Datenkabel
- 10: Lesegerät für Speichermedium
- 12: Schlüsselschalter
- 14: Drucktaster
- 16: Netzkabel
- 18: Hauptschalter

## Patentansprüche

1. Vorrichtung zur Aktivierung der Selbstheilungskräfte eines Patienten, umfassend
eine Bestrahlungseinheit (6) mit einer Anzahl Laserdioden für eine lichttherapeutische Behandlung;
eine Steuereinheit (4) zur Steuerung der Stärke und der Pulsfrequenzen der Laserdioden in der Bestrahlungseinheit; sowie
einen Prozessor zur Darstellung der physikalischen Parameter der Bestrahlung auf einem Monitor (2), **gekennzeichnet dadurch,**
**dass** der Prozessor ausgelegt ist für eine ärztliche Kommunikation und Interaktion mit dem Patienten und er die vorgesehene Behandlungsform auf dem Monitor durch Schrift- und Symbolzeichen sinnfällig für den Patienten darstellt,
**dass** die Bestrahlungseinheit bei der lichttherapeutischen Behandlung vom Patienten von Hand an den Ort der gewünschten therapeutischen Behandlung geführt werden kann; und
**dass** die zur Verfügung stehenden lichttherapeutischen Behandlungsformen für den Patienten im Prinzip unschädlich und ungefährlich sind;
so dass der Patient nach ärztlicher Anleitung an sich unüberwacht über einen längeren Zeitraum eine auf ihn individuell abstimmbare und veränderbare lichttherapeutische Behandlung kontrolliert vornehmen kann, während der er sich auf sich und bestimmte Körperpartien konzentriert und er hierdurch seine Selbstheilungskräfte aktiviert.

2. Vorrichtung gemäß Anspruch 1, wobei die Bestrahlungseinheit (6) drei unterschiedliche Laserdioden (6a, 6b, 6c) umfasst, welche bevorzugt Laserlicht mit Wellenlängen von 639 nm, 532 nm, beziehungsweise 405 nm produzieren.

3. Vorrichtung gemäß einem der Ansprüche 1 oder 2, wobei die Laserdioden mit Pulsfrequenzen zwischen 0,1 Hz und 1000 GHz strahlen können.

4. Vorrichtung gemäß einem der Ansprüche 1 bis 3, zudem umfassend ein Lesegerät (10) für ein Speichermedium.

5. Vorrichtung gemäß einem der Ansprüche 1 bis 4, wobei der Patient über die Steuereinheit (4) Therapieparameter, wie Dauer, Intensität, bevorzugte Laserdiode, Pulsfrequenz oder Bestrahlungsfläche, selbständig bestimmen kann.

6. Vorrichtung gemäß einem der Ansprüche 1 bis 5, wobei der Monitor (2) ein Bildschirm ist, der dem Patienten persönliche Daten und Behandlungsmethode wiedergibt, und/oder wobei der Monitor (2) ein berührungsempfindlicher Bildschirm ist, über den der Patient der Steuereinheit (4) der Vorrichtung Befehle erteilen kann.

7. Verwendung der Vorrichtung zur Aktivierung der Selbstheilungskräfte eines Patienten aus Ansprüchen 1 bis 6 zur Schmerzbehandlung, zur Behandlung von chronischen Krankheiten, wie amyotropher Lateralsklerose, Multipler Sklerose, Parkinson, Alzheimer, Gelenkkrankheiten, Diabetes, Rheuma, Krebs, AIDS (HIV), zur Behandlung von akuten Krankheiten, wie Kapselentzündungen, oder zur Behandlung von Verletzungen wie Knochenbrüche, Verbrennungen oder Muskelverletzungen.

8. Verwendung gemäß Anspruch 7, wobei mit der Bestrahlungseinheit (6) Hautpartien des Patienten bestrahlt werden.

9. Verwendung gemäß Anspruch 8, wobei mit der Bestrahlungseinheit (6) zudem eine Anzahl nicht-toxische Feststoffe, Flüssigkeiten oder Gase bestrahlt werden, die der Patient als Teil der Therapie zu sich nimmt, beziehungsweise inhaliert.

10. Verwendung gemäß Anspruch 9, wobei die Bestrahlung der Hautpartien und der Anzahl nicht-toxischer Feststoffe, Flüssigkeiten oder Gase nach dem gleichen Muster erfolgt.

11. Verfahren zur Aktivierung der Selbstheilungskräfte eines Patienten, umfassend die Bestrahlung von Hautpartien des Patienten mit elektromagnetischer Strahlung und/oder das Verabreichen von nicht-toxischen bestrahlten Feststoffen, Flüssigkeiten oder Gasen an den Patienten.

12. Verfahren zur Herstellung von Stoffen oder Gegenständen zur Aktivierung der Selbstheilungskräfte eines Patienten, umfassend das Bereitstellen eines Stoffes oder Gegenstands und die Bestrahlung des Stoffes oder Gegenstands mit elektromagnetischer Strahlung, wobei der Stoff oder Gegenstand fest, flüssig oder gasförmig sein kann, insbesondere ausgewählt aus Lebensmitteln, Tabletten, Globuli, Pflanzen, Pflanzenbestandteilen, Metallplatten, Steinen, Glas, Plexiglas, Kristallen, Wachs.

13. Verfahren gemäß Anspruch 12, wobei die Bestrahlung mit Hilfe eines Frequenzgenerators erfolgt, der wahlweise ein Laser, eine magnetische Spule, einen Zapper, ein Tens-Gerät, eine Elektroakupunkturvorrichtung, einen Biophotonenstrahler, eine Lakhovsky-Spirale, eine Tachyonenquelle oder eine beliebige andere Strahlenquelle ist.

14. Verfahren gemäß Anspruch 12, wobei die Aktivierung eines aktivierten Stoffes oder Gegenstands über einen Orgonstrahler auf einen nicht-aktivierten Stoff oder Gegenstand übertragen wird.

15. Stoff oder Gegenstand zur Aktivierung der Selbstheilungskräfte eines Patienten, hergestellt nach einem Verfahren gemäß einem der Ansprüche 12 bis 14.
